# EUROPEAN PATENT SPECIFICATION

(11) **EP 2 764 851 B1**
(45) Date of publication and mention of the grant of the patent: **06.11.2019**
(21) Application number: 14152086.6
(22) Date of filing: 22.01.2014
(51) Int. Cl.: A61F 2/44

(54) **STAGED EXPANSION VERTEBRA RETAINING DEVICE**
WIRBELHALTEVORRICHTUNG MIR STUFENWEISER AUSWEITUNG
DISPOSITIF DE RETENUE DE VERTÈBRE EN EXPANSION EN PLUSIEURS ÉTAPES

(30) Priority: 08.02.2013 TW 102105279
(43) Date of publication of application: 13.08.2014
(73) Proprietor: Chen, Kuei-Jung, Changua County 507 (TW)
(72) Inventor: Chen, Kuei-Jung, Changua County 507 (TW)
(74) Representative: Viering, Jentschura & Partner mbB Patent- und Rechtsanwälte

(56) References cited:
- US-A- 5 554 191
- US-A1- 2002 143 401
- US-A1- 2005 113 916
- US-A1- 2010 286 780

## Description

### BACKGROUND OF THE INVENTION

### 1. Field of the Invention

The present invention relates to a vertebra retaining device, and more particularly to a staged expansion vertebra retaining device for dispersing the applied force of the graft operation effectively. The vertebra retaining device is adjustable for the height of the vertebra, which can be adapted for most people. The vertebra retaining device is labor-saving, precise, and quick for a graft operation.

### 2. Description of the Prior Art

Due to age, accident, sports, poor posture for a long period, or occupational injuries, etc., the human spine is easy to appear spine dislocation, cartilage degradation, hyperostosis, particularly elderly degenerative spinal disease. Sometimes, it may cause serious complications, such as dysfunction of other organs of the body. In order to solve the degenerative diseases of the spine due to old age or other external forces, an intervertebral fusion surgery is required or an artificial intervertebral disk is grafted in between a pair of vertebral bodies.

TW Patent No. M414944, M411920, M409825, M400307 and M38401 disclose a vertebral graft instead of the original intervertebral disk of the human body. The vertebral graft is grafted in between two vertebral bodies or lumbar vertebras to provide a support and buffer function. However, the distance between two vertebral bodies is different. The vertebral graft has a fixed and single size, which may be too tight or loose when used for a graft operation.

U.S. Patent No. 5554191 discloses an intersomatic vertebral cage. As shown in the drawings, the intersomatic vertebral cage comprises a support main body 3 and a screw 36. The support main body 3 comprises two support portions 31, 32 having serrated surfaces 312, 313 thereon. The open ends of the two support portions 31, 32 have inclined guide surfaces 310, 320 to form a V-shaped opening 342. The screw 36 is inserted in the support main body 3. One end of the screw 36 extends out of the two support portions 31, 32 and is coupled with an expansion member 37. The expansion member 37 is normally against the two inclined guide surfaces 310, 320. When the intersomatic vertebral cage is grafted in between two vertebral bodies, the screw 36 is rotated by a tool 4 to expand the two support portions 31, 32 at an appropriate angle for holding the two vertebral bodies in place.

This structure has the angle expansion structure to solve the problem that the distance and size between two vertebral bodies are different. When it is used for a graft operation, the two support portions 31, 32 must be first expanded to hold two vertebral bodies, and then the screw is rotated continuously for expansion. However, both the two support portions 31, 32 are a one-piece structure. For the graft operation, it is necessary to apply a great force to expand the two support portions 31, 32 so as to hold the two vertebral bodies, namely, it is laborious for operation. During the graft operation, the intersomatic vertebral cage cannot be aligned and positioned precisely because of a slide caused by the excessive applied force. Sometimes, a readjustment is required, which increases the difficulty of the operation and extends the operation time and causes the patient a burden.

Accordingly, the inventor of the present invention has devoted himself based on his many years of practical experiences to solve this problem.

US 2010/0286780 A1 discloses an expendable spinal implant comprising a cage body including at least two movable branches having first end portions that are interconnected to one another and second end portions that are movable relative to one another. The movable branches include a first shell portion having a first pair of longitudinal edges and defining a first hollow region therebetween and a second shell portion having a second pair of longitudinal edges and defining a second hollow region therebetween with the first and second hollow region is cooperating to define at least a portion of a hollow interior of the cage body. An expansion member co-acts with the first and second shell portions to transition the cage body to an expanded configuration as the expansion member is actually displaced along the first and second pairs of longitudinal edges.

US 2005/0113916 A1 discloses an extended spinal planned including a body having a plurality of portions cooperating to define an outer cross-section having a first and a second Traverse dimension and the first and second substantially planar surface is disposed generally opposite to another and adapted to engage adjacent
vertebral bodies. And Nick member collects with the movable portions to expand the body along each of the first and second traverse dimensions.

US 2002/0143401 A1 discloses a staged expansion vertebra retaining device according to the preamble of claim 1. The staged expansion vertebra retaining device, wherein the staged expansion vertebra retaining device comprises a main body and a screw inserted into the main body, a distal end of the screw is provided with a push block, the main body has a transverse slot extending backward from a front end thereof to form at least two movable support units relative to one side of the push block, each movable support unit is split by a groove to form at least two movable support surfaces which are able to be expanded in a staged manner by the push block, so that the movable support units are expanded at an angle to hold two adjacent vertebral bodies in place, wherein, wherein an open end of the transverse slot is formed with an enlarged portion for the push block to be transversely disposed and screwed to the distal end of the screw.

### SUMMARY OF THE INVENTION

The primary object of the present invention is to provide a staged expansion vertebra retaining device for dispersing the applied force of the graft operation effectively. The present invention is adjustable for the height of the vertebra, which can be adapted for most people. The present invention is labor-saving, precise, and quick for a graft operation.

A further object of the present invention is to provide a staged expansion vertebra retaining device to shorten the graft operation time effectively.

The object of the invention is achieved by the subject matter of claim 1. Advantageous embodiments are disclosed by the dependent claims.

In order to achieve the aforesaid objects, a staged expansion vertebra retaining device is provided. The staged expansion vertebra retaining device of the present invention comprises a main body and a screw inserted into the main body. The distal end of the screw is provided with a push block. The main body has a traverse slot extending backward from a front end thereof to format least two movable support units relative to one side of the push block. Each movable support unit is split by a groove to form at least two movable support surfaces which are able to be expanded in a staged manner by the push block, so that the movable support units are expanded at an angle to hold two adjacent vertebral bodies in place. An open end of the transverse slot is formed with an enlarged portion for the push block to be transversely disposed and screwed to the distal end of the screw. The at least two movable surfaces comprise a movable support surface which is an innermost movable support surface and a frame shaped-moveable support surface which is an outermost movable support surface, and the inner surfaces of the movable support surfaces have at least two guide surfaces connecting the transverse slot and the enlarged portion and spaced at an interval for guiding the push block, by bringing the push block to move inward the first guide surface is pushed so that the innermost movable support surface is first expanded at an appropriate angle and when the push block is translated to move inward until the guide surface of the outermost movable support surface, the movable support surfaces are simultaneously expanded outward by the push block.

Preferably, the movable support surfaces have guide surfaces spaced at an interval for guiding the push block.

Preferably, the guide surfaces are inclined stepped guide surfaces.

Preferably, the guide surfaces are inclined guide surfaces.

Preferably, the guide surfaces are curved guide surfaces.

Preferably, the main body has an oblong shape. The upper and lower surfaces of the main body are formed with serrated surfaces. The main body has a transverse slot extending backward from a front end thereof to form the two movable support units relative to the upper and lower walls of the transverse slot. Each movable support unit is split by at least one groove to form a movable support surface and a frame-shaped movable support surface. One end of the movable support surface is connected with the inner edge of the frame-shaped movable support surface. An open end of the transverse slot is formed with an enlarged portion for the push block to be transversely disposed and screwed to the distal end of the screw. The inner surfaces of the movable support surface have guide surfaces connecting the transverse slot and the enlarged portion respectively. The guide surfaces are spaced at an interval. The guide surface of the innermost movable support surface is against the inner surface of the push block. When the screw is rotated to bring the push block to move inward, the push block is guided by the guide surfaces to expand the movable support surfaces in a staged manner, so that the movable support units are expanded at an angle to hold two adjacent vertebral bodies in place.

Preferably, the frame-shaped movable support surface is formed with a stop flange facing the open end of the transverse slot, such that the two stop flanges of the frame-shaped movable support surfaces of the two movable support units are adapted to stop the outer surface of the push block to prevent the push block from falling off outward.

Alternatively, the main body has a cylindrical shape or a hollow pillar shape having at least four sides. The outer surface of the main body is formed with a serrated surface. The main body has a split groove extending backward from a front end thereof to form at least two spaced movable support units. Each movable support unit is split by at least one groove to form at least one movable support surface and a frame-shaped movable support surface, the main body has a central axial hole. The front end of the axial hole is formed with an enlarged portion for a spherical push block to be accommodated and screwed to the distal end of the screw. The inner surfaces of the movable support surfaces have guide surfaces connecting the axial hole and the enlarged portion, respectively. The guide surfaces are spaced at an interval. The guide surface of the outermost frame-shaped movable support surface is at the forefront to be against the inner surface of the push block. When the screw is rotated to bring the push block to move inward, the push block is guided by the guide surfaces to expand the movable support surfaces in a staged manner, so that the movable support units disposed radially are expanded at an angle to hold two adjacent vertebral bodies in place.

Alternatively, the main body has an oblong shape. The upper and lower surfaces of the main body are formed with serrated surfaces. The main body has a transverse slot formed at a central section of a side wall thereof. The transverse slot has a dual-stepped shape having a wide outside and a narrow inside. The front end of the transverse slot is formed with an enlarged portion for the push block to be transversely disposed and screwed to the distal end of the screw. The upper and lower walls of the main body, relative to the transverse slot, are split by grooves to form two movable support units and two spaced upper and lower fixed frames. One end of the movable support surface is connected with the inner edge of the frame-shaped movable support surface. Each movable support unit is split by a groove to form at least two movable support surface which are gradually reduced from outside to inside. The inner surfaces of the movable support surface have guide surfaces connecting the transverse slot and the enlarged portion, respectively. The guide surfaces are spaced at an interval. The guide surface of the innermost movable support surface is at the forefront to be against the inner surface of the push block. When the screw is rotated to bring the push block to move inward, the upper and lower fixed frames are immovable and the push block is guided by the guide surfaces of the movable support units to expand the movable support surfaces in a staged manner, so that the movable support units disposed between the upper and lower fixed frames are expanded at an angle to hold two adjacent vertebral bodies in place.

Alternatively, the main body has a cylindrical shape or a hollow pillar shape having at least four sides. The surface of one end of the main body is split by grooves to form at least two spaced movable support units and a fixed frame. One end of the movable support surface is connected with the inner edge of the frame-shaped movable support surface. Each movable support unit is split by at least one groove to form at least two movable support surfaces which are gradually reduced from outside to inside. The main body has a central axial hole. The front end of the axial hole is formed with an enlarged portion for a spherical push block to be accommodated and screwed to the distal end of the screw. The inner surfaces of the movable support surfaces have guide surfaces connecting the axial hole and the enlarged portion, respectively. The guide surfaces are spaced at an interval. The guide surface of the outermost movable support surface is at the forefront to be against the inner surface of the push block. When the screw is rotated to bring the push block to move inward, the fixed frame is immovable and the push block is guided by the guide surfaces of the movable support surfaces to expand the movable support surfaces in a staged manner, so that the movable support units disposed radially are expanded at an angle to hold two adjacent vertebral bodies in place.

Compared to the prior art, the present invention provides the movable support surfaces having a staged expansion structure. When the screw is rotated to move the push block, one of the movable support surfaces is first expanded by the push block, and then the other movable support surfaces are continuously expanded at an appropriate angle. Thus, the applied force of the graft operation can be dispersed effectively. The present invention is adjustable for the height of the vertebra, which can be adapted for most people. The present invention is labor-saving, precise, and quick for a graft operation.

### BRIEF DESCRIPTION OF THE DRAWINGS

Fig. 1 is a perspective view accordingly to a first embodiment of the present invention;
Fig. 2 is a top view accordingly to the first embodiment of the present invention;
Fig. 3 is a schematic view of the first embodiment of the present invention coupled with the push block;
Figs. 4-6 are schematic views showing the expansion operation of the first embodiment of the present invention;
Fig. 7 is a schematic view showing the three-stage guide surfaces accordingly to the first embodiment of the present invention;
Fig. 8 is a schematic view showing the inclined guide surfaces accordingly to the first embodiment of the present invention;
Fig. 9 is a schematic view showing the inclined stepped guide surfaces accordingly to the first embodiment of the present invention;
Fig. 10 is a schematic view showing the curved guide surfaces accordingly to the first embodiment of the present invention;
Fig. 11 is a perspective view accordingly to a second embodiment of the present invention;
Fig. 12 is a sectional view accordingly to the second embodiment of the present invention;
Fig. 13 is a perspective view accordingly to a third embodiment of the present invention;
Fig. 14 is a sectional view accordingly to the third embodiment of the present invention;
Fig. 15 is a perspective view accordingly to a fourth embodiment of the present invention; and
Fig. 16 is a sectional view accordingly to the fourth embodiment of the present invention.

### DETAILED DESCRIPTION OF THE PREFERRED EMBODIMENTS

Embodiments of the present invention will now be described, by way of example only, with reference to the accompanying drawings.

The staged expansion vertebra retaining device of the present invention is made of titanium, tantalum, stainless steel, ceramics, aluminium oxide, PE, PU, PEEK, or silicon, which has better biocompatibility and mechanical property.

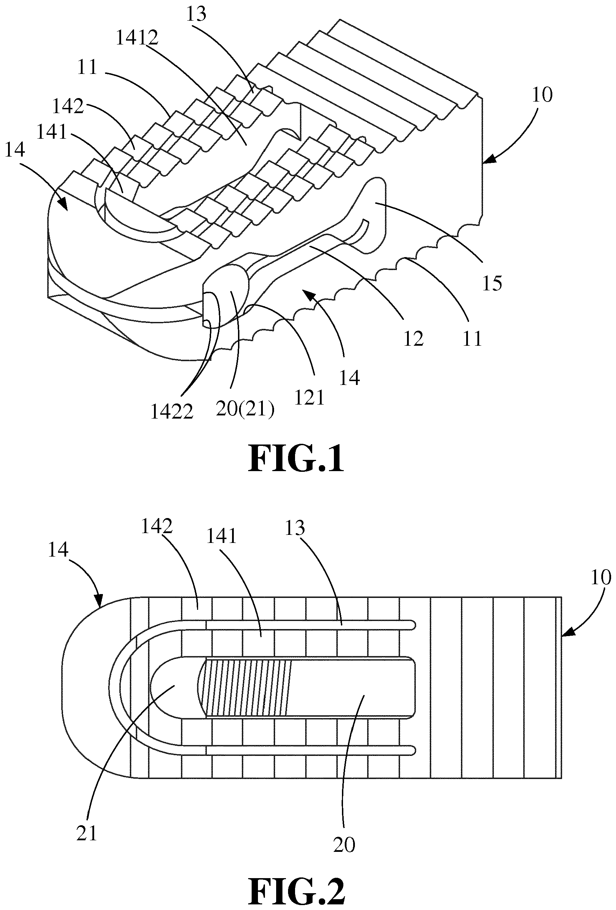

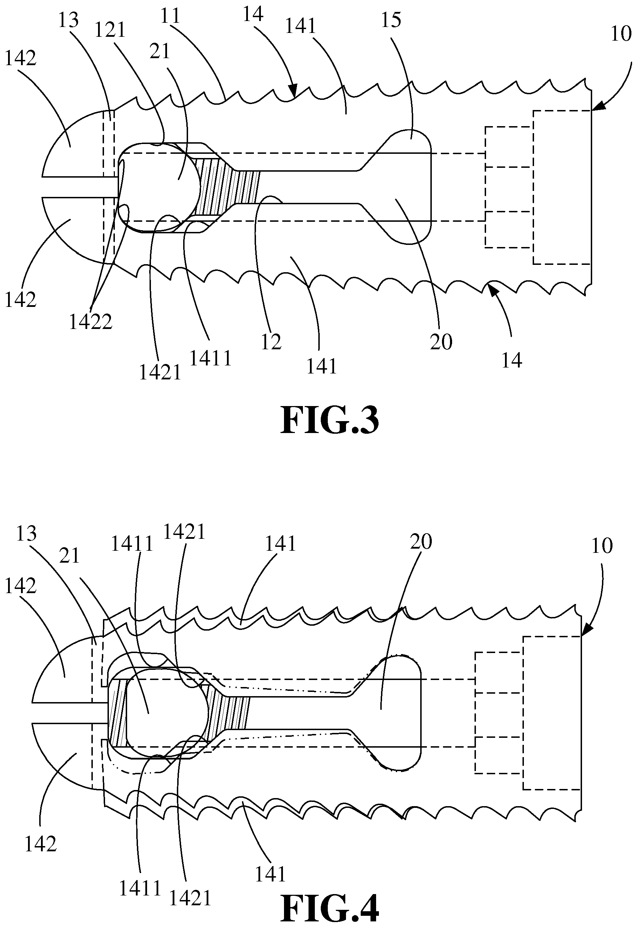

As shown in Fig. 1 to Fig. 6, the staged expansion vertebra retaining device according to a first embodiment of the present invention comprises a main body 10 and a screw 20 inserted into the main body 10. The distal end of the screw 20 is coupled with a push block 21. The main body 10 has an oblong shape. In the drawings, a lumbar vertebra graft is as an example, but not limited to. The upper and lower surfaces of the main body 10 are formed with serrated surfaces 11. The main body 10 has a transverse slot 12 extending backward from a front end thereof to form two spaced movable support units 14. Each movable support unit 14 is split by at least one groove 13 to form a movable support surface 141 and a frame-shaped movable support surface 142. The groove 13 and the movable support surface 141 have a U shape as shown in the drawings, but not limited to. One end of the movable support surface 141 is connected with an inner edge of the frame-shaped movable support surface 142. An open end of the transverse slot 12 is formed with an enlarged portion 121 for the push block 21 to be transversely disposed and screwed to the distal end of the screw 20. Referring to Fig. 3, the inner surfaces of the movable support surfaces 141, 142 have guide surfaces 1411, 1421 connecting the transverse slot 12 and the enlarged portion 121, respectively. The guide surfaces 1411, 1421 are spaced at an interval. As shown in the drawings, the guide surfaces 1411, 1421 are inclined surface, but not limited to. The guide surface 1411 of the innermost movable support surface 141 is against the inner surface of the push block 21. The frame-shaped movable support surface 142 is formed with a stop flange 1422 facing the open end of the transverse slot 12, such that the two stop flanges 1422 of the frame-shaped movable support surfaces 142 of the two movable support units 14 are adapted to stop the outer surface of the push block 21 to prevent the push block 21 from falling off outward. The inner end of the transverse slot 12 communicates with a vertical hole 15, so that it is more labor-saving for installation and expansion of the movable support surfaces 141, 142. Furthermore, the middle of the movable support surface 141 is formed with a middle hole 1412 for receiving autologous osteophyte and artificial bone issue during a surgical operation.

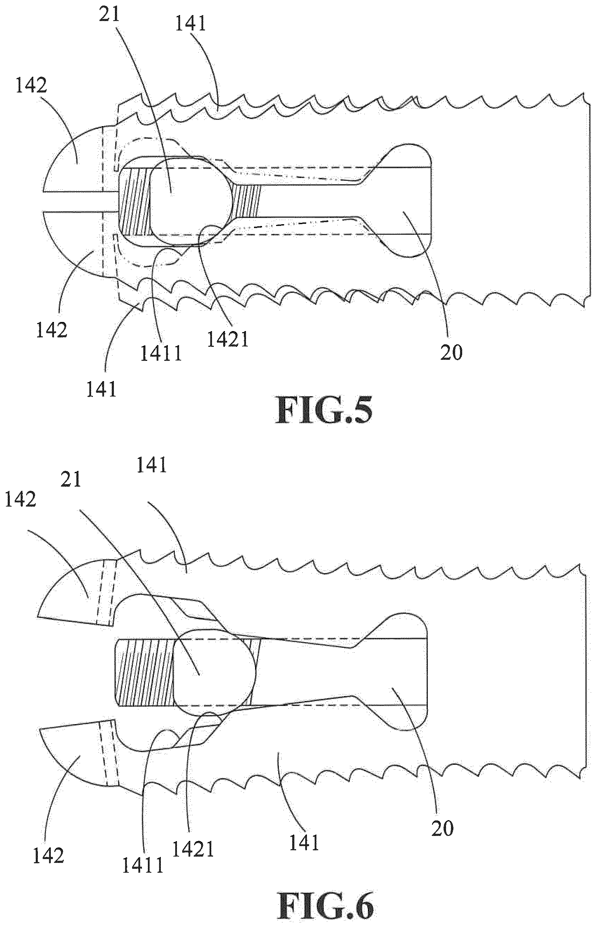

Referring to Fig. 4, when the present invention is installed, the push block 21 of the present invention is normally against the guide surface 1411 of the innermost movable support surface 141. When the present invention is grafted in between two vertebral bodies (not shown), the screw 20 is rotated by a tool (not shown) to bring the push block 21 to move inward and push the guide surface 1411 of the innermost movable support surface 141 so that the innermost movable support surface 141 is first expanded at an appropriate angle. Referring to Fig. 5, when the push block 21 is translated to move inward until the guide surface 1421 of the outermost movable support surface 142, the movable support surfaces 141, 142 are simultaneously expanded outward by the push block 21, as shown in Fig. 6. Thus, the movable support surfaces 141, 142 can be expanded fully in a staged manner from inside to outside. That is to say, the present invention uses the push block 21 to expand the innermost movable support surface 141 to hold two adjacent vertebral bodies (not shown) first. When it is expanded continuously until the predetermined route, the outermost movable support surface 142 will be expanded simultaneously. Through each movable support surface having a staged expansion structure, the operation force can be dispersed effectively. The graft operation can be first applied with an appropriate force, namely, the innermost movable support surface 141 is first to hold two adjacent vertebral bodies (not shown) to prevent the graft from sliding. The present invention is labor-saving, precise, and quick for a graft operation.

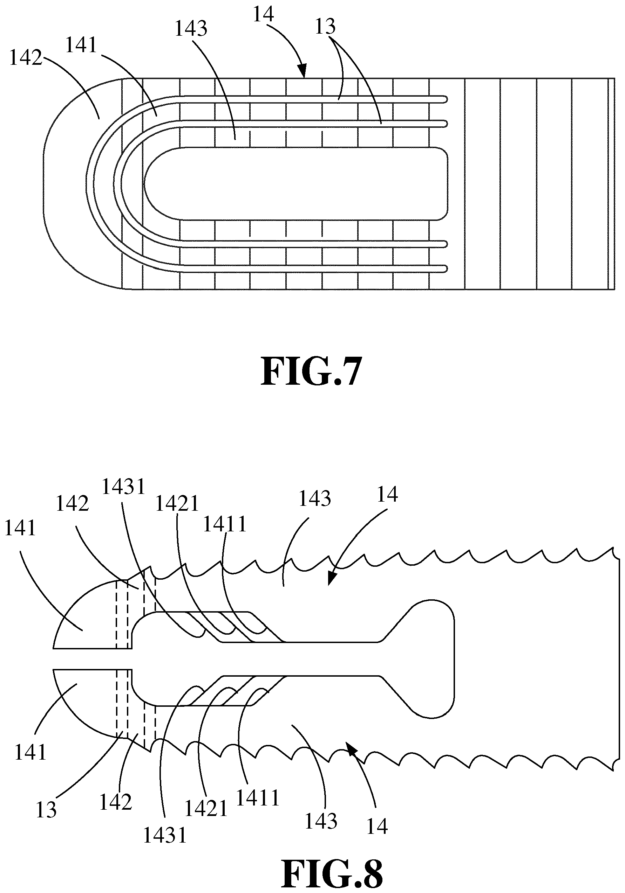

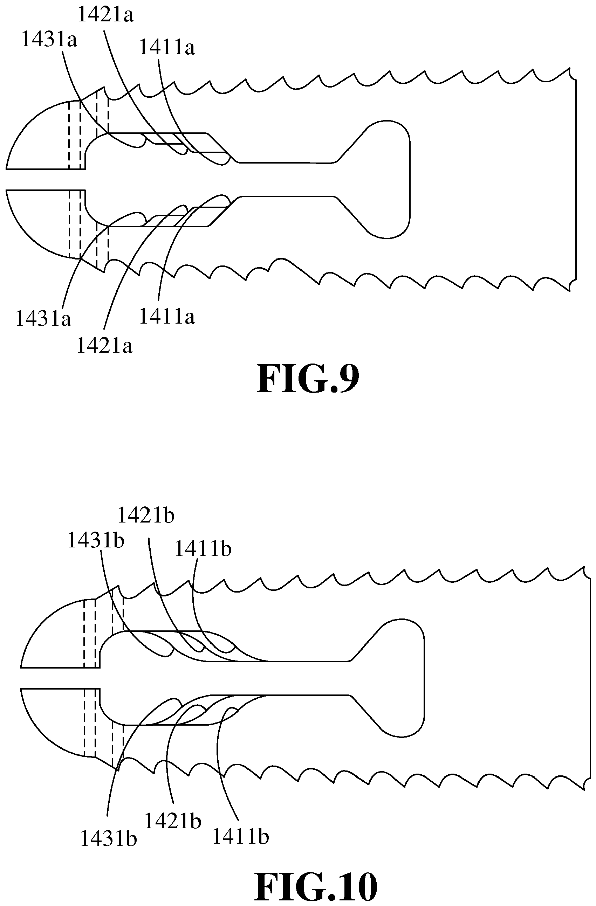

Referring to Fig. 7 and Fig. 8, each movable support unit 14 of the first embodiment of the present invention is split by the grooves 13 to form three movable support surfaces 142, 141, 143 which are disposed from outside to inside and gradually reduced to disperse the applied force in a three-stage manner. The guide surfaces 1421, 1411, 1431 at the open ends of the three movable support surfaces 142, 141, 143 can be inclined guide surfaces; or the guide surfaces 1421a, 1411a, 1431a can be inclined stepped guide surfaces, as shown in Fig. 9; or the guide surfaces 1421b, 1411b, 1431b can be curved guide surfaces, as shown in Fig. 10.

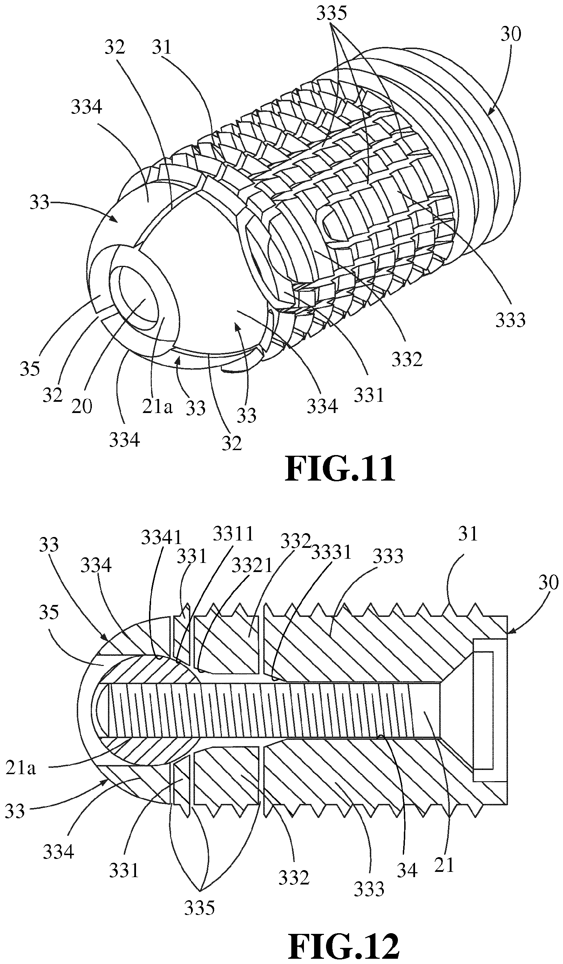

As shown in Fig. 11 to Fig. 12, the staged expansion vertebra retaining device according to a second embodiment of the present invention comprises a main body 30 and a screw 20 inserted into the main body 30. The distal end of the screw 20 is coupled with a spherical push block 21a. The main body 30 has a cylindrical shape or a hollow pillar shape having at least four sides. The outer surface of the main body 30 is formed with a serrated surface 31. The main body 30 has a split groove 32 extending backward from a front end thereof to form at least two spaced movable support units 33. Each movable support unit 33 is split by at least one groove 335 to form at least one movable support surface. In this embodiment, the main body 30 has three movable support surfaces 331, 332, 333 and a frame-shaped movable support surface 334. The groove 335 and the movable support surfaces 331, 332, 333 have a U shape as shown in the drawings, but not limited to. The main body 30 has a central axial hole 34. The front end of the axial hole 34 is formed with an enlarged portion 35 for the push block 21a to be accommodated and screwed to the distal end of the screw 20. The inner surfaces of the movable support surfaces 331, 332, 333, 334 have guide surfaces 3311, 3321, 3331, 3341 connecting the axial hole 34 and the enlarged portion 35, respectively. The guide surfaces 3311, 3321, 3331, 3341 are spaced at an interval, respectively. The guide surface 3341 of the outermost movable support surface 334 is at the forefront to be against the inner surface of the push block 21a. When the screw 20 is rotated to bring the push block 21a to move inward, the push block 21a is guided by the guide surfaces 3341, 3311, 3321, 3331 to expand the movable support surfaces 334, 331, 332, 333 in a staged manner, so that the movable support units 33 disposed radially are expanded at an appropriate angle to hold two adjacent vertebral bodies (not shown) in place.

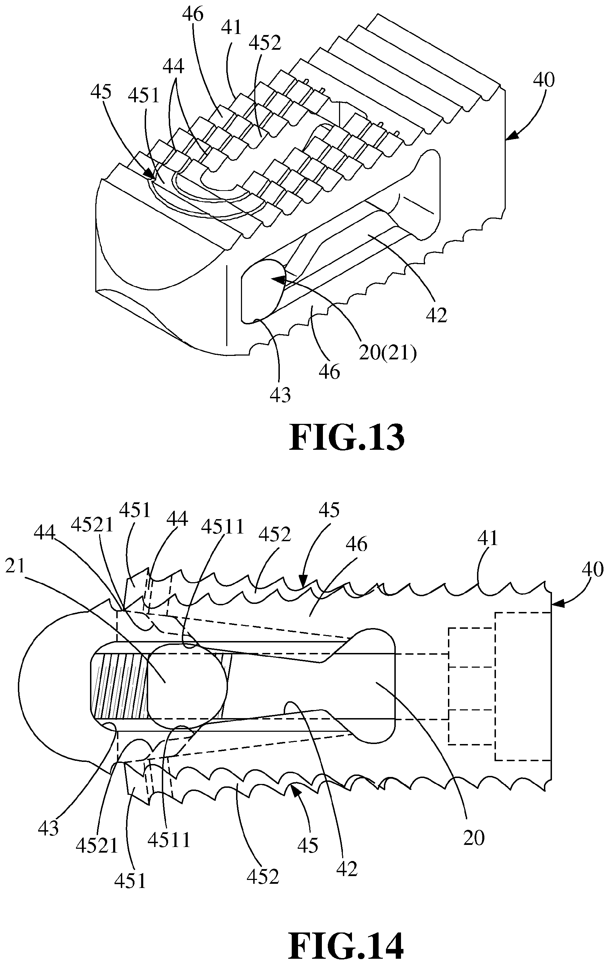

The present invention can keep the main body immobile, and only the movable support units disposed on the main body are expanded in between two vertebral bodies. As shown in Fig. 13 and Fig. 14, the staged expansion vertebra retaining device according to a third embodiment of the present invention comprises a main body 40 and a screw 20 inserted into the main body 40. The distal end of the screw 20 is coupled with a push block 21. The main body 40 has an oblong shape. The upper and lower surfaces of the main body 40 are formed with serrated surfaces 41. The main body 40 has a transverse slot 42 formed at a central section of a side wall thereof. The transverse slot 42 has a dual-stepped shape having a wide outside and a narrow inside. A front end of the transverse slot 12 is formed with an enlarged portion 43 for the push block 21 to be transversely disposed and screwed to the distal end of the screw 20. The upper and lower walls of the main body 40, relative to the transverse slot 40, are split by grooves 44 to form two movable support units 45 and two spaced upper and lower fixed frames 46. One end of each movable support unit 45 is connected with the inner edge of the corresponding fixed frame 46. Each movable support unit 45 is split by at least one groove 44 to form at least two movable support surface which are gradually reduced from outside to inside. As shown in the drawings, each movable support unit 45 has two movable support surfaces 451, 452. The movable support units 45, the movable support surface 451, 452, and the groove 44 have a U shape as shown in the drawings, but not limited to. The inner surfaces of the movable support surface 451, 452 have guide surfaces 4511, 4521 connecting the transverse slot 42 and the enlarged portion 43, respectively. The guide surfaces 4511, 4521 are spaced at an interval. The guide surface 4521 of the innermost movable support surface 452 is at the forefront to be against the inner surface of the push block 21. When the screw 20 is rotated to bring the push block 21 to move inward, the upper and lower fixed frames 46 are immovable and the push block 21 is guided by the guide surfaces 4511, 4521 of the movable support units 451, 452 to expand the movable support surfaces 451, 452 in a staged manner, so that the movable support units 45 disposed between the upper and lower fixed frames 46 are expanded at an appropriate angle to hold two adjacent vertebral bodies (not shown) in place.

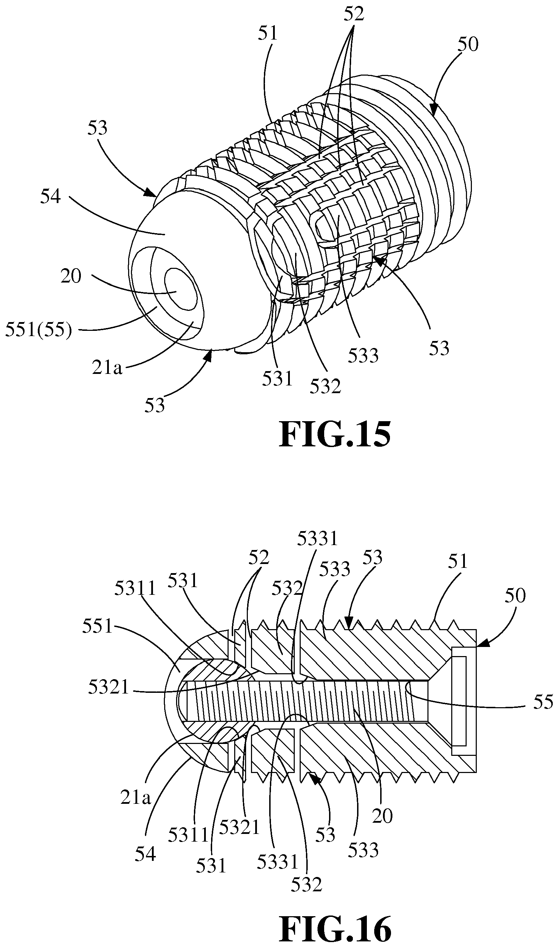

As shown in Fig. 15 to Fig. 16, the staged expansion vertebra retaining device according to a fourth embodiment of the present invention comprises a main body 50 and a screw 20 inserted into the main body 50. The distal end of the screw 20 is coupled with a spherical push block 21a. The main body 50 has a cylindrical shape or a hollow pillar shape having at least four sides. The outer surface of the main body 50 is formed with a serrated surface 51. The surface of one end of the main body 50 is split by a groove 52 to form at least two spaced movable support units 53. In this embodiment, the main body 50 has three movable support units 53 and a fixed frame 54. One end of each movable support unit 53 is connected with the inner edge of the fixed frame 54. Each movable support unit 53 is split by the same groove 52 to form at least two movable support surfaces. In this embodiment, the main body 50 has three movable support surfaces 531, 532, 533. The movable support units 53, the movable support surfaces 531, 532, 533 and the groove 52 have a U shape as shown in the drawings, but not limited to. The main body 50 has a central axial hole 55. The front end of the axial hole 55 is formed with an enlarged portion 551 for the push block 21a to be accommodated and screwed to the distal end of the screw 20. The inner surfaces of the movable support surfaces 531, 532, 533 have guide surfaces 5311, 5321, 5331 connecting the axial hole 55 and the enlarged portion 551, respectively. The guide surfaces 5311, 5321, 5331 are spaced at an interval, respectively. The guide surface 5311 of the outermost movable support surface 531 is at the forefront to be against the inner surface of the push block 21a. When the screw 20 is rotated to bring the push block 21a to move inward, the fixed frame 54 is immovable and the push block 21a is guided by the guide surfaces 5311, 5321, 5331 of the movable support surfaces 531, 532, 533 to expand the movable support surfaces 531, 532, 533 in a staged manner, so that the movable support units 53 disposed radially are expanded at an appropriate angle to hold two adjacent vertebral bodies (not shown).

To sum up the aforesaid embodiments, the present invention provides the movable support surfaces having a staged expansion structure. When the screw is rotated to move the push block, one of the movable support surfaces is first expanded by the push block, and then the other movable support surfaces are continuously expanded at an appropriate angle. Thus, the applied force of the graft operation can be dispersed effectively. The present invention is adjustable for the height of the vertebra, which can be applied to most people. The present invention is labor-saving, precise, and quick for a graft operation.

Although particular embodiments of the present invention have been described in detail for purposes of illustration, various modifications and enhancements may be made without departing from the present invention. Accordingly, the present invention is not to be limited except as by the appended claims.

## Claims

1. A staged expansion vertebra retaining device, wherein the staged expansion vertebra retaining device comprises a main body (10) and a screw (20) inserted into the main body (10), a distal end of the screw (20) is provided with a push block (21), the main body (10) has a transverse slot (12) extending backward from a front end thereof to form at least two movable support units (14) relative to one side of the push block (21), each movable support unit (14) is split by a groove (13) to form at least two movable support surfaces (141, 142) which are able to be expanded in a staged manner by the push block (21), so that the movable support units (14) are expanded at an angle to hold two adjacent vertebral bodies in place, wherein an open end of the transverse slot (12) is formed with an enlarged portion (121) for the push block (21) to be transversely disposed and screwed to the distal end of the screw (20), **characterized in that**
the at least two movable support surfaces (141, 142) comprise a movable support surface (141) which is an innermost movable support surface (141) and a frame-shaped moveable support surface (142) which is an outermost movable support surface (142), and the inner surfaces of the movable support surfaces (141, 142) have at least two guide surfaces (1411, 1421) connecting the transverse slot (12) and the enlarged portion (121) and spaced at an interval for guiding the push block (21), by bringing the push block (21) to move inward the first guide surface (1411) is pushed so that the innermost movable support surface (141) is first expanded at an appropriate angle and when the push block (21) is translated to move inward until the guide surface (1421) of the outermost movable support surface (142), the movable support surfaces (141, 142) are simultaneously expanded outward by the push block (21).

2. The staged expansion vertebra retaining device as claimed in claim 1, wherein the guide surfaces (1411a, 1421a) are inclined stepped guide surfaces.

3. The staged expansion vertebra retaining device as claimed in claim 1, wherein the guide surfaces (1411, 1421) are inclined guide surfaces.

4. The staged expansion vertebra retaining device as claimed in claim 1, wherein the guide surfaces (1411b, 1421b) are curved guide surfaces.

5. The staged expansion vertebra retaining device as claimed in any one of claims 1-4, wherein the main body (10) has an oblong shape, upper and lower surfaces of the main body (10) are formed with serrated surfaces (11), the two movable support units (14) being formed relative to upper and lower walls of the transverse slot (12), the guide surface (1411) of the innermost movable support surface (141) is against an inner surface of the push block (21), when the screw (20) is rotated to bring the push block (21) to move inward, the guide surfaces (1411, 1421) are expanded in a staged manner, so that the movable support units (14) are expanded at an angle to hold two adjacent vertebral bodies in place.

6. The staged expansion vertebra retaining device as claimed in claim 5, wherein the frame-shaped movable support surface (142) is formed with a stop flange (1422) facing the open end of the transverse slot (12), such that two stop flanges (1422) of the frame-shaped movable support surfaces (142) of the two movable support units (14) are adapted to stop an outer surface of the push block (21) to prevent the push block (21) from falling off outward.

7. The staged expansion vertebra retaining device as claimed in any one of claims 1-4, wherein the main body (30) has a cylindrical shape or a hollow pillar shape having at least four sides, an outer surface of the main body (30) is formed with a serrated surface (31), the main body (30) has a central axial hole (34), a front end of the axial hole (34) is formed with an enlarged portion (35) for the push block (21a) to be accommodated, wherein the push block (21a) is spherical, wherein the guide surfaces (3311, 3341) connect the axial hole (34) and the enlarged portion (35) respectively, the guide surface (3341) of the outermost frame-shaped movable support surface (334) is at the forefront to be against an inner surface of the push block (21a), when the screw (20) is rotated to bring the push block (21a) to move inward, the guide surfaces (3341, 3311) are expanded in a staged manner, so that the movable support units (33) disposed radially are expanded at an angle to hold two adjacent vertebral bodies in place.

## Patentansprüche

1. Eine gestuft ausweiterbare Wirbelhaltevorrichtung, wobei die gestuft ausweiterbare Wirbelhaltevorrichtung einen Hauptteil (10) und eine in den Hauptteil (10) eingesetzte Schraube (20) umfasst; am distalen Ende der Schraube (20) ein Druckblock (21) vorgesehen ist, der Hauptteil (10) einen Querschlitz (12) aufweist, der sich von dessen vorderen Ende nach hinten erstreckt, um mindestens zwei bewegliche Unterstützungseinheiten (14) zu bilden, die relativ zu einer Seite des Druckblocks (21) angeordnet sind; jede bewegliche Unterstützungseinheit (14) durch eine Nut (13) in mindestens zwei bewegliche Tragflächen (141, 142) geteilt wird, die mit einem Druckblock (21) gestuft ausgeweitet werden können, um die beweglichen Unterstützungseinheiten (14) in einen Winkel auszuweiten und somit zwei angrenzende Wirbelkörper vor Ort festzuhalten, während ein offenes Ende des Querschlitzes (12) mit einem vergrößerten Teil (121) gebildet ist, um den Druckblock (21) quer anzuordnen und an das distale Ende der Schraube (20) anzuschrauben, **dadurch gekennzeichnet, dass**
die mindestens zwei beweglichen Tragflächen (141, 142) eine bewegliche Tragfläche (141) umfassen, die eine innerste bewegliche Tragfläche (141) und eine bewegliche Tragfläche (142) in Rahmenform ist, die eine äußerste bewegliche Tragfläche (142) ist, wobei die Innenflächen der beweglichen Tragflächen (141, 142) mindestens zwei Führungsflächen (1411, 1421) aufweisen, mit denen der Querschlitz (12) und der vergrößerte Teil (121) verbunden und in einem Abstand angeordnet sind, um den Druckblock (21) zu führen; durch Bewegen des Druckblocks (21) nach innen die erste Führungsfläche (1411) angeschoben wird, damit die innerste bewegliche Tragfläche (141) zuerst in einem geeigneten Winkel ausgeweitet wird, wobei beim Übertragen des Druckblocks (21) dieser nach innen bis zur Führungsfläche (1421) der äußerten beweglichen Tragfläche (142) bewegt wird, wobei die beweglichen Tragflächen (141, 142) mit dem Druckblock (21) gleichzeitig nach außen bewegt werden.

2. Die gestuft ausweiterbare Wirbelhaltevorrichtung nach Anspruch 1, wobei die Führungsflächen (1411a, 1421a) als geneigte und stufige Führungsflächen gebildet sind.

3. Die gestuft ausweiterbare Wirbelhaltevorrichtung nach Anspruch 1, wobei die Führungsflächen (1411, 1421) als geneigte Führungsflächen gebildet sind.

4. Die gestuft ausweiterbare Wirbelhaltevorrichtung nach Anspruch 1, wobei die Führungsflächen (1411b, 1421b) als gebogene Führungsflächen gebildet sind.

5. Die gestuft ausweiterbare Wirbelhaltevorrichtung nach einem der Ansprüche 1-4, wobei der Hauptteil (10) eine längliche Form aufweist; die oberen und unteren Oberflächen des Hauptteils (10) mit gezackten Oberflächen (11) gebildet sind; die zwei beweglichen Unterstützungseinheiten (14) relativ zu den oberen und unteren Wänden des Querschlitzes (12) gebildet sind; die Führungsfläche (1411) der innersten beweglichen Tragfläche (141) gegen eine Innenfläche des Druckblocks (21) andrückt; beim Rotieren der Schraube (20), um den Druckblock (21) nach innen zu bewegen, die Führungsflächen (1411, 1421) gestuft ausgeweitet werden, so dass die beweglichen Unterstützungseinheiten (14) in einem Winkel ausgeweitet werden, um zwei anstoßende Wirbelkörper vor Ort festzuhalten.

6. Die gestuft ausweiterbare Wirbelhaltevorrichtung nach Anspruch 5, wobei die bewegliche Tragfläche (142) in Rahmenform gegenüber dem offenen Ende des Querschlitzes (12) mit einem Anschlagflansch (1422) gebildet ist, so dass die zwei Anschlagflansche (1422) der beweglichen Tragflächen (142) in Rahmenform der beweglichen Unterstützungseinheiten (14) zum Anhalten einer Außenfläche des Druckblocks (21) geeignet sind, um ein Hinausfallen des Druckblocks (21) zu vermeiden.

7. Die gestuft ausweiterbare Wirbelhaltevorrichtung nach einem der Ansprüche 1-4, wobei der Hauptteil (30) eine zylindrische oder hohle Kissenform mit mindestens vier Seiten aufweist; eine Außenfläche des Hauptteils (30) mit einer gezackten Oberfläche (31) gebildet ist; der Hauptteil (30) eine Axialbohrung (34) in der Mitte aufweist; ein vorderes Ende der Axialbohrung (34) mit einem vergrößerten Teil (35) gebildet ist, um den Druckblock (21a) aufzunehmen, wobei der Druckblock (21a) kugelförmig ist; wobei die Führungsflächen (3311, 3341) die Axialbohrung (34) bzw. den vergrößerten Teil (35) verbinden; die Führungsfläche (3341) der äußersten beweglichen Tragfläche (334) in Rahmenform auf der Vorderseite ist, um an eine Innenfläche des Druckblocks (21a) anzudrücken; beim Rotieren der Schraube (20), um den Druckblock (21a) nach innen zu bewegen, die Führungsflächen (3341, 3311) auf eine gestufte Weise ausgeweitet werden, damit die bewegliche Unterstützungseinheiten (33) radial in einem Winkel ausgeweitet werden, um die beiden angrenzenden Wirbelkörper vor Ort festzuhalten.

## Revendications

1. Dispositif de retenue de vertèbre en expansion en plusieurs étapes, **caractérisé par le fait que** le dispositif de retenue de vertèbre en expansion en plusieurs étapes comprend un corps principal (10) et une vis (20) insérée dans le corps principal (10), une extrémité distale de la vis (20) est munie d'un bloc poussoir (21), le corps principal (10) comporte une fente transversale (12) s'étendant vers l'arrière à partir de son extrémité avant pour former au moins deux unités de support mobiles (14) par rapport à un côté du bloc poussoir (21), chaque unité de support mobile (14) est divisée par une rainure (13) pour former au moins deux surfaces de support mobiles (141, 142) qui peuvent être étendues de manière progressive par le bloc poussoir (21), de sorte que les unités de support mobiles (14) sont étendues à un angle pour maintenir en place deux corps vertébraux adjacents, une extrémité ouverte de la fente transversale (12) est formée avec une partie élargie (121) pour que le bloc poussoir (21) soit disposé transversalement et vissé sur l'extrémité distale de la vis (20),
**caractérisé en ce que**
les deux, ou plus, surfaces de support mobiles (141, 142) comprennent une surface de support mobile (141) qui est une surface de support mobile la plus proche de l'intérieur (141) et une surface de support mobile en forme de cadre (142) qui est une surface de support mobile la plus proche de l'extérieur (142), et les surfaces internes des surfaces de support mobiles (141, 142) comportent au moins deux surfaces de guidage (1411, 1421) reliant la fente transversale (12) et la partie élargie (121) et espacées à un intervalle pour guider le bloc poussoir (21), en amenant le bloc poussoir (21) à se déplacer vers l'intérieur, la première surface de guidage (1411) est poussée de sorte que la surface de support mobile la plus proche de l'intérieur (141) est d'abord étendue à un angle approprié et lorsque le bloc poussoir (21) est transféré pour se déplacer vers l'intérieur jusqu'à ce que la surface de guidage (1421) de la surface de support mobile la plus proche de l'extérieur (142), les surfaces de support mobiles (141, 142) sont simultanément étendues vers l'extérieur par le bloc poussoir (21).

2. Dispositif de retenue de vertèbre en expansion en plusieurs étapes selon la revendication 1, **caractérisé par le fait que** les surfaces de guidage (1411a, 1421a) sont des surfaces de guidage à étages inclinés.

3. Dispositif de retenue de vertèbre en expansion en plusieurs étapes selon la revendication 1, **caractérisé par le fait que** les surfaces de guidage (1411, 1421) sont des surfaces de guidage inclinées.

4. Dispositif de retenue de vertèbre en expansion en plusieurs étapes selon la revendication 1, **caractérisé par le fait que** les surfaces de guidage (1411b, 1421b) sont des surfaces de guidage incurvées.

5. Dispositif de retenue de vertèbre en expansion en plusieurs étapes selon l'une quelconque des revendications 1-4, **caractérisé par le fait que** le corps principal (10) comporte une forme oblongue, les surfaces supérieure et inférieure du corps principal (10) sont formées de surfaces dentelées (11), les deux unités de support mobiles (14) étant formées par rapport aux parois supérieure et inférieure de la fente transversale (12), la surface de guidage (1411) de la surface de support mobile la plus proche de l'intérieur (141) est contre une surface intérieure du bloc poussoir (21), lorsque la vis (20) est tournée pour amener le bloc poussoir (21) à se déplacer vers l'intérieur, les surfaces de guidage (1411, 1421) sont étendues de manière progressive, de sorte que les unités de support mobiles (14) sont étendues à un angle pour maintenir en place deux corps vertébraux adjacents.

6. Dispositif de retenue de vertèbre en expansion en plusieurs étapes selon la revendication 5, **caractérisé par le fait que** la surface de support mobile en forme de cadre (142) est formée avec une bride de butée (1422) à l'opposé de l'extrémité ouverte de la fente transversale (12), de sorte que deux brides de butée (1422) des surfaces de support mobile en forme de cadre (142) des deux unités de support mobiles (14) sont adaptées pour arrêter une surface extérieure du bloc poussoir (21) pour empêcher le poussoir de bloc (21) de tomber vers l'extérieur.

7. Dispositif de retenue de vertèbre en expansion en plusieurs étapes selon l'une quelconque des revendications 1-4, **caractérisé par le fait que** le corps principal (30) comporte une forme cylindrique ou une forme de pilier creux ayant au moins quatre côtés, une surface extérieure du corps principal (30) est formé avec une surface dentelée (31), le corps principal (30) comporte un trou axial central (34), une extrémité avant du trou axial (34) est formé avec une partie élargie (35) pour accueillir le bloc poussoir (21a), le bloc poussoir (21a) est sphérique, les surfaces de guidage (3311, 3341) relient le trou axial (34) et la partie élargie (35) respectivement, la surface de guidage (3341) de la surface de support mobile en forme de cadre la plus proche de l'extérieur (334) est en première ligne pour être contre une surface intérieure du bloc poussoir (21a), lorsque la vis (20) est tournée pour amener le bloc poussoir (21a) à se déplacer vers l'intérieur, les surfaces de guidage (3341, 3311) sont étendues de manière progressive, de sorte que les unités de support mobiles (33) disposées radialement sont étendues à un angle pour maintenir en place deux corps vertébraux adjacents.
